# FASCICULE DE BREVET EUROPEEN

(11) **EP 3 658 249 B1**
(45) Date de publication et mention de la délivrance du brevet: **27.10.2021**
(21) Numéro de dépôt: 18749024.8
(22) Date de dépôt: 03.07.2018
(51) Int. Cl.: C07C 67/03, C07C 67/54, C07C 213/06, C07C 213/10, C07C 69/54, C07C 219/08, B01D 3/14

(54) **PROCEDE DE PURIFICATION D'ESTERS (METH)ACRYLIQUES**
VERFAHREN ZUR REINIGUNG VON (METH)ACRYLSÄUREESTERN
METHOD FOR PURIFYING (METH)ACRYLIC ESTERS

(30) Priorité: 25.07.2017 FR 1757038
(43) Date de publication de la demande: 03.06.2020
(73) Titulaire: Arkema France, 92700 Colombes (FR)
(72) Inventeur: ROLEZ, Eloi, 57000 Metz (FR); TRETJAK, Serge, 57520 Roulhing (FR)
(74) Mandataire: Arkema Patent
(86) Numéro de dépôt international: PCT/FR2018/051645
(87) Numéro de publication internationale: WO 2019/020889

(56) Documents cités:
- EP-A2- 2 659 943
- WO-A1-2012/071158
- JP-A- 2005 239 564
- US-A1- 2016 158 667
- ASPRION N ET AL: "Dividing wall columns: Fundamentals and recent advances", CHEMICAL ENGINEERING AND PROCESSING, ELSEVIER SEQUOIA, LAUSANNE, CH, vol. 49, no. 2, 25 janvier 2010 (2010-01-25), pages 139-146, XP026938678, ISSN: 0255-2701, DOI: 10.1016/J.CEP.2010.01.013 [extrait le 2010-01-25] cité dans la demande
- DEJANOVIC I ET AL: "Dividing wall column-A breakthrough towards sustainable distilling", CHEMICAL ENGINEERING AND PROCESSING, ELSEVIER SEQUOIA, LAUSANNE, CH, vol. 49, no. 6, 12 avril 2010 (2010-04-12) , pages 559-580, XP027138843, ISSN: 0255-2701 [extrait le 2010-04-12] cité dans la demande

## Description

### DOMAINE TECHNIQUE

La présente invention concerne la production d'esters (méth)acryliques selon un procédé en continu par transestérification, et en particulier la purification d'un mélange réactionnel brut comprenant un ester (méth)acrylique en C₄-C₁₂ à l'aide d'une colonne à partition mise en œuvre dans une configuration particulière.

Cette configuration conduit à une simplification du procédé de purification avec une consommation énergétique réduite et une teneur minimisée en impuretés présentes dans l'ester (méth)acrylique purifié.

L'invention a trait également à un procédé de production d'ester (méth)acrylique en C₄-C₁₂, comprenant ce procédé de récupération/purification.

### ARRIERE-PLAN TECHNIQUE ET PROBLEME TECHNIQUE

La synthèse d'esters (méth)acryliques en C₄-C₁₂ est généralement effectuée par réaction de transestérification entre un (méth)acrylate d'alcool léger (dénommé (méth)acrylate léger) et un alcool lourd (alcool en C₄-C₁₂ correspondant).

Cette réaction est une réaction catalysée équilibrée avec génération d'alcool léger qu'il est nécessaire d'éliminer au cours de la réaction pour déplacer l'équilibre dans le sens de la production de l'ester (méth)acrylique.

Les réactions secondaires au cours de la synthèse produisent des impuretés, généralement des sous-produits de point d'ébullition élevé ou proche du point d'ébullition de l'ester recherché, qu'il est nécessaire d'éliminer en vue d'obtenir l'ester (méth)acrylique avec une pureté élevée, satisfaisant aux exigences techniques liées à son utilisation finale en tant que monomère pour fabriquer des polymères utilisables dans de nombreux domaines d'application.

Par ailleurs, les produits valorisables présents dans le mélange réactionnel, notamment les réactifs non réagis et le catalyseur, sont dans la mesure du possible recyclés au sein du procédé.

A ces fins, on procède généralement à un processus de séparation/purification comportant un ensemble de distillations, d'extractions et/ou décantations, qui est à la fois relativement complexe à mettre en œuvre, et coûteux d'un point de vue énergétique, et investissement pour un atelier industriel.

Des procédés de purification d'esters (méth)acryliques ont été largement décrits dans l'art antérieur.

On peut citer par exemple le document US 7,268,251 dans lequel l'effluent réactionnel provenant d'une transestérification est traité par un procédé nécessitant la mise en œuvre d'au moins quatre colonnes de distillation ou de rectification, dont un évaporateur pour séparer le catalyseur. Il s'avère que le procédé décrit dans le document US 7,268,251 est compliqué à mettre en œuvre à l'échelle industrielle, du fait de l'optimisation des conditions opératoires de la succession des quatre éléments de distillation / rectification , pour obtenir un produit d'une grande pureté et une production satisfaisante. Ce procédé, très coûteux en investissement nécessite en outre une surface d'implantation importante. Il est illustré avec la fabrication d'acrylate de diméthyl aminoéthyle à partir de diméthyl aminoéthanol et d'acrylate d'éthyle.

Le document EP 960 877 au nom de la Société déposante, illustre un autre procédé de fabrication d'acrylate de diméthyl aminoéthyle. Ce procédé consiste en une élimination du catalyseur et des produits lourds (équeutage) suivie d'une élimination des composés légers (étêtage) et d'une rectification finale d'un mélange brut réactionnel. Ce procédé présente ainsi l'avantage de ne comporter que trois colonnes de distillation dans le train de purification du mélange réactionnel.

Le document US 7,294,240 B2 décrit un procédé de fabrication en continu d'esters (méth)acryliques, en particulier la fabrication d'acrylate de diméthyl aminoéthyle à partir de diméthyl aminoéthanol et d'acrylate de butyle, en combinant une synthèse réalisée dans un système réactionnel comprenant deux réacteurs en série afin de limiter les réactions secondaires, une colonne permettant de séparer le catalyseur du reste du mélange réactionnel et une colonne à distiller à soutirage latéral permettant d'obtenir un acrylate de diméthyl aminoéthyle de haute pureté. La purification du mélange réactionnel met en œuvre deux colonnes de distillation et l'ester purifié est soutiré latéralement de la seconde colonne.

Le document WO 2013/110876 décrit un procédé de purification adapté en particulier à l'acrylate de 2-octyle, comprenant une séparation préalable du catalyseur et la purification à l'aide de deux colonnes de distillation en série ou une colonne de distillation à soutirage latéral.

Il a par ailleurs été proposé d'utiliser une seule colonne de distillation à soutirage latéral pour purifier en une seule étape un mélange réactionnel d'acrylate d'alkyle en C₄-C₉, comme décrit dans le document WO 2014/096648 au nom de la Société déposante.

De façon générale, les procédés de l'art antérieur associent une première séparation du catalyseur du milieu réactionnel, puis une distillation du mélange réactionnel exempt de catalyseur à l'aide d'une ou deux colonnes à distiller pour obtenir un ester (méth)acrylique de grande pureté.

Des procédés de purification simplifiés sont proposés grâce au développement des colonnes de distillation à partition (connues sous la dénomination DWC- Divided wall column). Cette technologie est basée sur une colonne de distillation comprenant une paroi interne de séparation ce qui permet de combiner le fonctionnement de deux colonnes traditionnellement en série dans un seul appareillage.

A titre d'exemple, la demande de brevet EP 2 659 943 décrit une configuration d'une colonne à partition et son fonctionnement dans un procédé de production d'acrylate de 2-éthylhexyle de haute pureté. Bien que cette colonne soit complexe à fabriquer et à opérer, elle présente l'avantage de réduire le coût d'équipement et la consommation d'énergie du processus de purification, comparativement à une installation conventionnelle comprenant deux colonnes de distillation. La question de la stabilisation nécessaire à son bon fonctionnement n'est pas abordée. Le procédé de purification décrit dans ce document ne fait pas mention des problèmes liés à la séparation préalable du catalyseur dont la présence peut engendrer des réactions de rétrogradation dans la colonne à partition.

La demande de brevet JP 2005-239564 décrit également l'utilisation d'une colonne à partition dans un procédé de synthèse d'esters (méth)acryliques, exemplifiée dans le cas de la synthèse de méthacrylate de butyle par réaction de transestérification entre le méthacrylate de méthyle et le butanol. Dans ce procédé, un dévésiculeur est associé à la colonne à partition pour éviter l'entraînement de gouttelettes de stabilisants dans le soutirage latéral et contrôler la quantité de stabilisants dans le produit purifié. La colonne à partition permet d'effectuer la séparation de l'ester visé avec les produits lourds et les produits plus légers. Cependant, le procédé de purification décrit dans ce document applicable à la production de (méth)acrylates d'amino alkyle et ne résout pas la question de la séparation préalable du catalyseur lorsque les esters sont sensibles à des réactions de rétrogradation. En particulier, la présence du catalyseur dans la colonne à partition risque d'engendrer des réactions de craquage conduisant à la formation de composés polluant le produit purifié soutiré latéralement.

La technologie des colonnes à partition en général est connue, et décrite par exemple par Asprion N et al, « Dividing wall columns : Fundamentals and recent advances », Chemical Engineering and processing, vol 49 (2010) pages 139-146 ; ou par Dejanovic I et al, « Dividing wall column - A breakthrough towards sustainable distilling », Chemical Engineering and processing, vol 49 (2010) pages 559-580.

Cependant, les différentes configurations décrites concernent uniquement le gain énergétique attendu, et l'état de la technique dans son ensemble ne suggère nullement le type de configuration adapté à la purification de mélanges réactionnels (méth)acryliques bruts issus d'une réaction de transestérification catalytique.

Il subsiste encore un besoin d'améliorer la purification des esters (méth)acryliques « lourds », comme par exemple l'acrylate de diméthyl aminoéthyle ou l'acrylate de 2-octyle, en termes de séparation / recyclage du catalyseur, et de bilan énergétique du procédé global de purification.

L'objectif de la présente invention répond à ce besoin en fournissant un procédé de récupération d'un ester (méth)acrylique en C₄-C₁₂ purifié à l'aide d'un système de purification comprenant une colonne à partition mise en œuvre dans une configuration particulière, qui permet d'éviter le risque de pollution de l'ester produit fini par les réactions de rétrogradation en présence de catalyseur dans la colonne.

La présente invention fournit ainsi une solution technico-économique au problème de la purification d'un mélange réactionnel brut résultant de la réaction de transestérification d'un ester léger d'acide (méth)acrylique par un alcool en C₄-C₁₂, avec une réduction du coût d'investissement et d'installation lié à une diminution du nombre de colonnes et de matériels connexes (pompes, échangeurs,...), mais aussi avec un gain énergétique de mise en œuvre, tout en répondant à la demande en matière de pureté de l'ester produit.

### RESUME DE l'INVENTION

L'invention a pour objet un procédé de récupération d'un ester (méth)acrylique en C₄-C₁₂ purifié, à partir d'un mélange réactionnel brut obtenu par transestérification d'un ester (méth)acrylique léger par l'alcool correspondant, ledit mélange réactionnel brut comprenant un catalyseur de transestérification,
ledit procédé étant caractérisé en ce qu'il est mis en œuvre à l'aide d'un système de purification comprenant une colonne à partition équipée d'une paroi séparatrice créant des zones de séparation dans la colonne, la paroi étant non jointive avec le dôme supérieur de la colonne en partie haute et jointive avec le fond de la colonne en partie basse, ladite colonne à partition associée en tête à un condenseur unique et en pied à deux bouilleurs, comprenant une section commune de rectification au-dessus de la paroi, une section de préfractionnement comportant l'alimentation de la colonne, une section de soutirage séparée de la section de préfractionnement par la paroi comprenant le soutirage de l'ester purifié, et
caractérisé en ce que i) un flux gazeux est extrait en tête de la section de rectification et recyclé après condensation au moins en partie dans le réacteur, ii) un flux est soutiré en pied de la section de préfractionnement et recyclé au moins en partie dans le réacteur, iii) un flux est soutiré en pied de la section de soutirage et recyclé au moins en partie dans la section de préfractionnement de la colonne, et iv) un flux d'ester (méth)acrylique purifié est soutiré latéralement de la section de soutirage en un point situé au-dessus du soutirage de pied de ladite section de soutirage.

Selon l'invention, le mélange réactionnel brut soumis au procédé de récupération de l'ester (méth)acrylique purifié, comprend au moins une partie, de préférence la totalité, du catalyseur mis en œuvre pour la réaction de transestérification.

Le procédé selon l'invention s'applique à la synthèse de (méth)acrylates d'alkyle, l'alcool utilisé dans la réaction de transestérification étant un alcool aliphatique primaire ou secondaire, comportant une chaine alkyle linéaire ou ramifiée comportant de 4 à 12 atomes de carbone, de préférence de 5 à 10 atomes de carbone, et pouvant être interrompue par un ou plusieurs hétéroatomes tels que N ou O, de préférence N.

Selon un mode de réalisation de l'invention, l'alcool est un alcool aliphatique primaire ou secondaire, à chaine alkyle linéaire ou ramifiée comportant de 4 à 12 atomes de carbone, de préférence de 5 à 10 atomes de carbone.

Comme exemples d'alcool, on peut citer le 2-éthyl hexanol, le 2-octanol, ou le 2-propyl heptanol. De préférence, l'alcool est le 2-octanol.

Selon un mode de réalisation de l'invention, l'alcool est un amino alcool, en particulier un dialkyl amino alcool, de formule :

HO-A- N (R₁)(R₂)

dans laquelle
- A est un radical alkylène, linéaire ou ramifié, en C₁-C₅
- R₁ et R₂, identiques ou différents l'un de l'autre, représentent chacun un radical alkyle en C₁-C₄.

Comme exemples d'alcool, on peut citer le N,N-diméthyl aminoéthanol (DMAE), le N,N-diéthyl aminoéthanol, le N,N-diméthyl aminopropanol.

De préférence, l'alcool est le N,N-diméthyl aminoéthanol (DMAE), dénommé aussi dans la suite de l'exposé par diméthyl aminoéthanol.

Par ester (méth)acrylique léger, on entend un (méth)acrylate à chaine alkyle en C₁ ou C₂, tel que le (méth)acrylate de méthyle ou le (méth)acrylate d'éthyle.

Le terme « (méth)acrylique » signifie acrylique ou méthacrylique ; le terme « (méth)acrylate » signifie acrylate ou méthacrylate.

De préférence, l'ester (méth)acrylique léger est l'acrylate de méthyle ou l'acrylate d'éthyle, plus préférentiellement l'acrylate d'éthyle.

Selon un mode de réalisation préféré, l'ester (méth)acrylique en C₄-C₁₂ purifié est un acrylate en C₄-C₁₂ purifié, plus préférentiellement l'acrylate de 2-octyle ou l'acrylate de diméthyl aminoéthyle (ADAME).

Le procédé de récupération selon l'invention conduit à un (méth)acrylate en C₄-C₁₂ de pureté au moins équivalente à celle obtenue dans une installation conventionnelle comprenant au moins deux colonnes de séparation, et cela dans des conditions de fonctionnement qui minimisent la dégradation thermique des composés thermosensibles, et dans des conditions énergétiques plus économiques.

Un autre objet de l'invention est l'utilisation d'un système de purification pour récupérer un ester (méth)acrylique en C₄-C₁₂ purifié, à partir d'un mélange réactionnel brut obtenu par transestérification catalytique d'un ester (méth)acrylique léger par l'alcool correspondant, ledit système comprenant une colonne à partition équipée d'une paroi séparatrice créant des zones de séparation dans la colonne, la paroi étant non jointive avec le dôme supérieur de la colonne en partie haute et jointive avec le fond de la colonne en partie basse, ladite colonne à partition associée en tête à un condenseur unique et en pied à deux bouilleurs, comprenant une section commune de rectification au-dessus de la paroi, une section de préfractionnement comportant l'alimentation de la colonne en un mélange à purifier, une section de soutirage séparée de la section de préfractionnement par la paroi comprenant le soutirage du produit purifié.

Un autre objet de l'invention est un procédé de production d'un ester (méth)acrylique en C₄-C₁₂ purifié, par transestérification d'un ester (méth)acrylique léger avec l'alcool correspondant, caractérisé en ce que le mélange réactionnel brut est soumis au procédé de récupération à l'aide du système de purification tel que défini ci-dessus.

Ainsi, l'invention permet d'atteindre les spécifications souhaitées en terme de pureté des esters (méth)acryliques dans des conditions économiques.

### BREVE DESCRIPTION DES FIGURES

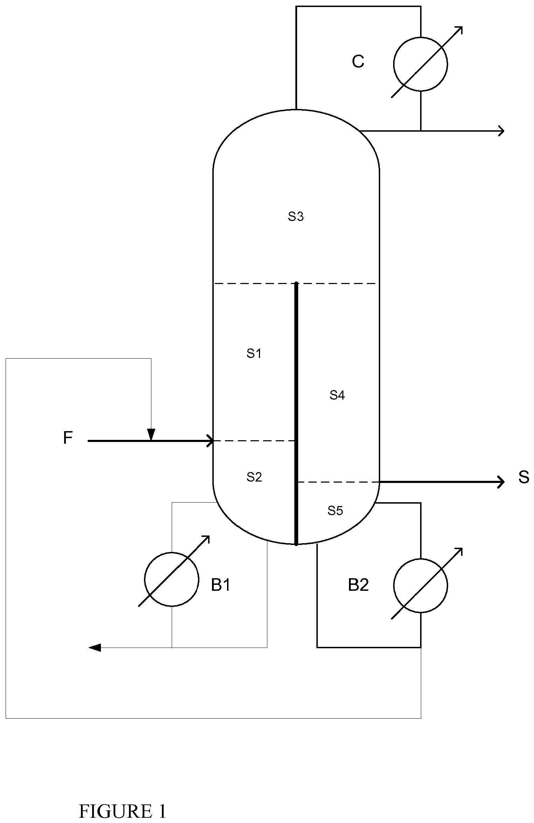

La Figure unique représente la configuration d'un système de purification comprenant une colonne à partition utilisable dans le procédé selon l'invention.

### EXPOSE DETAILLE DE L'INVENTION

L'invention est maintenant décrite plus en détail et de façon non limitative dans la description qui suit.

Par souci de simplicité de la suite de l'exposé, et de façon non limitative, le procédé de l'invention est décrit en référence à la production d'acrylate de diméthyl aminoéthyle (ADAME) obtenu par transestérification à partir d'acrylate d'éthyle (AE) et diméthyl aminoéthanol (DMAE).

Dans le réacteur de transestérification, les composés légers présents dans le milieu réactionnel sont généralement les réactifs résiduels - l'acrylate d'éthyle et le diméthyl aminoéthanol dans ce cas particulier.

Une réaction de cyclisation peut conduire à la formation de 1,4-diméthyl pipérazine (DMPPZ) dont le point d'ébullition est proche de celui du diméthyl aminoéthanol. Ce sous-produit va donc être extrait en même temps que l'alcool résiduel.

Au titre des réactions secondaires conduisant à la formation de sous-produits lourds au cours de la synthèse d'esters (méth)acryliques, il existe notamment une réaction d'addition de Michael d'une molécule contenant un atome d'hydrogène labile (tel qu'un alcool) sur la double-liaison d'un composé (méth)acrylique (tel que le (méth)acrylate léger ou l'ester (méth)acrylique formé). Des réactions de cyclisation peuvent aussi se produire.

Dans le cas particulier de la fabrication de l'ADAME à partir d'AE et DMAE, l'alcool DMAE n'ayant pas encore réagi ou l'éthanol libéré s'ajoutent à la double liaison de l'ADAME déjà formé ou de l'AE n'ayant pas réagi, pour former des sous-produits lourds d'addition de Michael [DMAE+ADAME] ou [DMAE+AE].

Une caractéristique de ces sous-produits est que leur point d'ébullition se trouve au-dessus des points d'ébullition des produits mis en œuvre dans la réaction et/ou de l'ADAME recherché. C'est pourquoi, l'ensemble de ces sous-produits est appelé par la suite sous-produits lourds.

Dans le cas particulier de la synthèse de l'ADAME, les sous-produits lourds comprennent des adduits de Michael, notamment l'éthoxy propionate d'éthyle (EPE), l'éthoxy propianate d'amiéthyle (EPA), l'amiétoxy propionate d'amiéthyle (APA)et l'amiétoxy propionate d'éthyle (APE), et divers autres composés lourds.

Le procédé selon l'invention a pour objectif de récupérer l'ester (méth)acrylique avec une pureté supérieure à 99,5%, de préférence supérieure à 99,8% à partir du mélange réactionnel comprenant des sous-produits légers (composés volatils ou composés légers), des sous-produits lourds (composés lourds ou composés les moins volatils), ainsi que le catalyseur de transestérification, et les inhibiteurs de polymérisation généralement ajoutés dans le réacteur de transestérification. Ceci peut être réalisé avec un système de purification comprenant une colonne à paroi séparatrice associée à un condenseur en tête et deux bouilleurs en pied, tel que représenté à la Figure annexée.

En référence à la Figure, la colonne à partition utilisée dans le procédé selon l'invention comporte une paroi (ou cloison) verticale partielle P, placée à l'intérieur de la colonne définissant ainsi trois zones distinctes : une zone supérieure, dénommée section de rectification, une zone centrale comprenant deux zones de part et d'autre de la cloison et s'étendant jusqu'au bas de la colonne.

Selon un mode de réalisation, la paroi peut être en partie diagonale. La paroi peut être plane ou cylindrique de sorte que les espaces séparés par la paroi peuvent être disposés sous forme concentrique.

La paroi telle que mise en place ne sépare pas forcément la zone centrale en deux zones égales, il peut en effet être avantageux dans certains modes de réalisation d'avoir des zones inégales afin de minimiser la perte de charge ou la tendance à l'engorgement selon la nature ou l'intensité des flux circulant dans la colonne.

La hauteur de la paroi représente de 30 à 70 % de la hauteur de la colonne.

La zone centrale est constituée de deux zones de part et d'autre de la paroi, dont l'une représente une section dite de préfractionnement et l'autre une section de soutirage du produit pur.

Par souci de simplification dans la suite de l'exposé de l'invention, par « section de préfractionnement », on entend la section de la colonne à paroi séparatrice qui est alimentée par le flux d'ester (méth)acrylique à purifier, l'alimentation ne se faisant que d'un seul côté de la paroi ; et par « section de soutirage », on entend la section de la colonne de l'autre côté de la paroi séparatrice d'où est extrait latéralement le flux d'ester (méth)acrylique purifié.

La section de préfractionnement associée à un bouilleur B1, comporte l'alimentation F de la colonne, séparant ainsi une section S1 au-dessus de l'alimentation et une section S2 au-dessous de l'alimentation. La section de préfractionnement a pour effet de concentrer les produits les plus volatils dit composés légers, en tête de la colonne, et de concentrer les produits les moins volatils dits composés lourds en pied de la colonne. C'est en particulier dans cette section de préfractionnement associée au bouilleur B1 que l'on retrouve en pied de celle-ci le catalyseur, une grande partie des inhibiteurs de polymérisation ainsi que les impuretés lourdes. Ce pied de préfractionnement peut être valorisé en recyclant tout ou partie de celui-ci dans le réacteur de transestérification.

Selon un mode de réalisation, l'alimentation se situe à la moitié inférieure de la section de préfractionnement, de préférence au tiers inférieur, par exemple sous le dernier plateau.

La section de soutirage comporte une sortie latérale afin de soutirer l'ester purifié S, la sortie latérale divisant la section de soutirage en deux sections S4 et S5. Le soutirage de l'ester purifié peut être réalisé sous forme d'un flux liquide ou d'un flux gazeux, de préférence on soutire un flux gazeux. Dans cette section, les composés légers ainsi que l'ester sont envoyés en tête de la colonne et les composés lourds sont envoyés en pied de la colonne. Un flux de pied comprenant essentiellement des composés lourds et des inhibiteurs de polymérisation et un peu d'ester produit, est soutiré de la section de soutirage associée à un bouilleur B2, et est recyclé avantageusement, au moins en partie, dans la section de préfractionnement, de préférence au niveau de l'alimentation F, ou en un point situé au-dessus ou en-dessous de l'alimentation. Le recyclage du pied de la section de soutirage permet de minimiser les pertes en ester (méth)acrylique.

Selon un mode de réalisation, le point de soutirage latéral se situe dans la moitié inférieure de la section de soutirage, de préférence au quart inférieur.

Au-dessus de la paroi en tête de la colonne à partition se trouve une zone commune, dite section de rectification S3 qui permet de séparer les composés légers qui sont extraits, puis condensés au moins en partie, dans le condenseur C associé à la colonne. Ce produit condensé est renvoyé en partie comme reflux dans la section S3, l'autre partie étant envoyé avantageusement au moins en partie à l'entrée du réacteur puisqu'il est constitué principalement de réactifs non réagis et un peu d'ester formé

Un reflux liquide en tête de colonne, peut ainsi être assuré. Au-dessus de la paroi, le liquide est récupéré et séparé de part et d'autre de la paroi vers les sections S1 et S4. La fraction massique de liquide retournant vers la section S1 est généralement comprise entre 20 et 50%.

Un certain nombre de paramètres caractérisent le design et le fonctionnement de la colonne à partition. Il s'agit principalement du nombre d'étages théoriques dans chaque section de la colonne à partition, en particulier les nombres N1, N2, N3, N4, N5 correspondant respectivement au nombre d'étages de chacune des sections S1 à S5 décrites précédemment, du taux de reflux de la colonne, du rapport de flux liquide provenant de la section de rectification de chaque côté de la paroi, du rapport de flux gazeux provenant de la section de rebouillage de chaque côté de la cloison, du positionnement du point d'alimentation F ou du point de soutirage latéral S du produit pur.

Ces différents paramètres peuvent être déterminés à partir de méthodes connues par l'homme de l'art de façon à ce que l'ester (méth)acrylique soit produit avec une pureté répondant aux spécifications désirées.

La colonne à partition et les internes présents sont choisis pour obtenir le nombre d'étages théoriques nécessaires dans chaque section. On pourra utiliser comme internes des plateaux, du garnissage ordonné comme du garnissage structuré ou du garnissage en vrac.

Selon un mode de réalisation, le nombre d'étages théoriques de la section de préfractionnement S1+S2 est compris entre 1 et 20, et l'alimentation de la colonne est de préférence placée au dernier tiers inférieur environ de cette section.

Selon un mode de réalisation, le nombre d'étages théoriques de la section de soutirage S4+S5 est compris entre 1 et 15, et le point de soutirage de l'ester purifié est de préférence placé au dernier quart inférieur environ de cette section.

Selon un mode de réalisation, le nombre d'étages théoriques de la section de rectification S3 est compris entre 1 et 15.

La colonne peut opérer sous vide, afin de minimiser l'exposition thermique des composés thermosensibles au sein de la colonne. Avantageusement, la colonne fonctionne sous un vide allant de 10 à 100 mm de Hg (1,33 à 13, 33 kPa).

Avantageusement la température de fonctionnement est comprise entre 50°C et 150 °C.

Les internes utilisés pour la colonne peuvent être des plateaux à clapets ou des plateaux perforés à déversoir, ou du garnissage structuré.

Hormis des conditions opératoires adaptées pour la réaction de transestérification minimisant la formation des composés lourds et optimisant le rendement de la réaction, il est nécessaire d'introduire des inhibiteurs de polymérisation (dénommés aussi stabilisants) non seulement au cours de la réaction, mais également au cours de la purification du mélange réactionnel brut sortant du réacteur d'estérification.

Les inventeurs ont découvert que la stabilisation du système de purification combinant une colonne à partition est plus avantageuse que la stabilisation d'une installation conventionnelle comprenant deux colonnes en série. En effet, l'inhibiteur de polymérisation utilisé pour stabiliser l'ester recherché peut être introduit dans le système de purification comme inhibiteur de polymérisation unique, il s'ensuit une simplification et une constance de la stabilisation. En alternative, un inhibiteur de polymérisation moins onéreux peut être utilisé pour stabiliser la colonne à partition, et l'ester purifié est ensuite stabilisé avec un autre composé plus adapté pour stabiliser le produit fini en vue de son stockage et utilisation ultérieure. Dans ce cas, le coût lié aux inhibiteurs de polymérisation peut être fortement réduit.

Comme inhibiteurs de polymérisation utilisables, on peut citer par exemple la phénothiazine (PTZ), l'hydroquinone (HQ), l'éther mono méthylique d'hydroquinone (EMHQ), le diterbutyl para-crésol (BHT), la paraphénylène diamine, le TEMPO (2,2,6,6-tétraméthyl-1-piperidinyloxy), le di-tertiobutylcatéchol, ou les dérivés du TEMPO, tel que le OH-TEMPO, seuls ou leurs mélanges en toutes proportions.

Selon un mode de réalisation, la stabilisation du système de purification est réalisée à l'aide d'un inhibiteur de polymérisation unique, de préférence injecté au niveau du condenseur de tête, l'ester (méth)acrylique purifié étant soutiré latéralement de la colonne à partition sous forme d'un flux liquide ou gazeux stabilisé.

Selon ce mode de réalisation, on préfère utiliser l'éther mono méthylique d'hydroquinone comme stabilisant.

Selon un mode de réalisation, la stabilisation du système de purification est réalisée à l'aide d'un premier inhibiteur de polymérisation, de préférence injecté au niveau du condenseur de tête, l'ester (méth)acrylique purifié étant soutiré latéralement de la colonne à partition sous forme d'un flux gazeux qui, après condensation, est ensuite stabilisé avec un inhibiteur de polymérisation différent du premier inhibiteur. Selon ce mode de réalisation, il est possible d'utiliser un premier inhibiteur nettement moins cher et s'affranchir de sa présence dans le produit purifié en effectuant un soutirage en phase gazeuse, le premier inhibiteur de polymérisation restant dans le flux de sous-produits lourds séparé en pied de colonne. La phénothiazine ou le OH-TEMPO peuvent convenir comme premier inhibiteur de polymérisation car ils permettent également de stabiliser l'ensemble des flux organiques. Le produit purifié soutiré latéralement est alors stabilisé, après condensation, selon la pratique conventionnelle, par exemple à l'aide d'éther méthylique d'hydroquinone.

Avantageusement, on introduit de 100 à 5000 ppm d'inhibiteur de polymérisation lors de la purification du mélange réactionnel dans le système de purification selon le procédé de l'invention.

Pour rendre les inhibiteurs plus efficaces, il est possible d'injecter en pied de colonne de l'oxygène, de l'air ou de l'air dit appauvri par exemple à 7% O₂. De façon préférée la quantité d'oxygène injectée correspond à une teneur de 0,2% à 0,5% vol. rapportée à la quantité de vapeur organique dans la colonne

Par ester (méth)acrylique purifié, on entend un produit ayant une teneur en ester (méth)acrylique > 99,5 % en poids, de préférence > 99,8% en poids, et généralement une teneurs en impuretés lourdes inférieure à 1500 ppm, avantageusement inférieure à 1200 ppm.

L'invention a aussi pour objet un procédé de production d'un ester (méth)acrylique en C₄-C₁₂ purifié, par transestérification caractérisé en ce que le mélange réactionnel brut est soumis au procédé de récupération à l'aide d'un système de purification tel que défini précédemment.

Les conditions de la réaction de transestérification sont celles connues par l'homme de l'art, et peuvent être mises en œuvre selon un procédé de type continu, semi-continu ou discontinu.

L'invention fournit ainsi un procédé de production d'un ester (méth)acrylique en C₄-C₁₂ dans une installation compacte dont le coût d'investissement et de fonctionnement est réduit, et fournissant un produit de haute pureté avec un rendement optimisé.

Par rapport aux procédés de l'art antérieur, il est possible de réduire de plus de 10% la consommation énergétique pour mettre en œuvre le procédé de purification selon l'invention. En outre, la teneur en impuretés difficiles à séparer du fait de leur point d'ébullition proche de celui de l'ester recherché, se trouve minimisée dans le produit purifié obtenu selon le procédé selon l'invention.

Les exemples ci-après illustrent la présente invention sans toutefois en limiter la portée.

### PARTIE EXPERIMENTALE

Dans les exemples, les pourcentages sont indiqués en poids sauf indication contraire et les abréviations suivantes ont été utilisées :
AE : Acrylate d'éthyle
APA : amiétoxy propionate d'amiéthyle
EPA : éthoxy propionate d'amiéthyle
EPE : éthoxy propionate d'éthyle
ADAME : acrylate de diméthyl aminoéthyle
PTZ : phénothiazine
DMAE : diméthyl aminoéthanol
EMHQ : éther méthylique d'hydroquinone
Catalyseur : exprimé sous forme de titanate d'éthyle

### Exemple 1 (comparatif) : Purification à l'aide de 3 colonnes de distillation en série

Un mélange réactionnel brut d'ADAME issu de la synthèse par transestérification d'acrylate d'éthyle et de diméthyl aminoéthanol a été soumis à un traitement de purification à l'aide de trois colonnes de distillation en série.

La première colonne comporte un équivalent en étages théoriques de 9 et elle est associée en pied à un bouilleur, et en tête à un condenseur dans lequel une phase organique est recyclée pour partie dans la colonne afin d'assurer un reflux de celle-ci. La colonne est stabilisée par injection de PTZ au niveau du condenseur de tête. L'énergie fournie par le bouilleur est 0,74 MW.

La seconde colonne comporte un équivalent en étages théoriques de 14 et elle est associée en pied à un bouilleur et en tête à un condenseur. Elle est alimentée par le flux de pied de la première colonne qui comprend l'ADAME, ainsi que les produis légers (AE, DMAE) et des sous-produits lourds tels que les adduits de Michael. La seconde colonne est stabilisée par injection de PTZ au niveau du condenseur de tête. L'énergie fournie par le bouilleur est 0,61 MW.

La troisième colonne comporte un équivalent en étages théoriques de 4 et elle est associée en pied à un bouilleur et en tête à un condenseur. Elle est alimentée par le flux de pied de la deuxième colonne qui comprend l'ADAME, des sous-produits lourds tels que les adduits de Michael et les stabilisants. La troisième colonne est stabilisée par injection d'EMHQ au niveau du condenseur de tête. L'énergie fournie par le bouilleur est 0,36 MW. L'ADAME purifié distille en tête de la troisième colonne.

L'alimentation de la première colonne a la composition massique et les caractéristiques suivantes :
ADAME : 48% - AE : 23% - DMAE : 14%
APA : 1% - EPA : 0,2% - EPE : 0,04% - Autres lourds : 164 ppm, catalyseur : 1,5%
Flux total : 6780 kg/h - température : 110 °C - Pression : 0,891 bar

Une simulation ASPEN à l'aide d'un modèle thermodynamique a été réalisée et fournit la composition massique suivante pour le produit purifié distillé en tête de la troisième colonne.
ADAME : 99,8% - DMAE : < 1 ppm
APA traces - EPA : 214 ppm - EPE : 889 ppm - Lourds dont EPA : 729 ppm
Flux total : 2870 kg/h - Température : 35°C - Pression : 0,05 bar (5 kPa)

Dans cette configuration, l'ADAME avec une pureté supérieure à 99,8% est récupéré avec un rendement de l'ordre de 88% par rapport au flux d'alimentation. La consommation énergétique globale est de 1,7 MW. La somme des impuretés lourdes (EPE, EPA, autres lourds) s'élève à 1618 ppm.

### Exemple 2 (comparatif) : Purification à l'aide de 2 colonnes de distillation en série et soutirage latéral du produit purifié

Un mélange réactionnel brut d'ADAME issu de la synthèse par transestérification d'acrylate d'éthyle et de diméthyl aminoéthanol a été soumis à un traitement de purification à l'aide de deux colonnes de distillation en série.

La première colonne comporte un équivalent en étages théoriques de 9 et elle est associée en pied à un bouilleur, et en tête à un condenseur dans lequel une phase organique est recyclée pour partie dans la colonne afin d'assurer un reflux de celle-ci. La colonne est stabilisée par injection de PTZ au niveau du condenseur de tête. L'énergie fournie par le bouilleur est 0,74 MW.

La seconde colonne comporte un équivalent en étages théoriques de 16 et elle est associée en pied à un bouilleur et en tête à un condenseur. Elle est alimentée par le flux de pied de la première colonne qui comprend l'ADAME, ainsi que les produis légers (AE, DMAE) et des sous-produits lourds tels que les adduits de Michael. La seconde colonne est stabilisée par injection de EMHQ au niveau du condenseur de tête. L'énergie fournie par le bouilleur est 0,8MW. Cette seconde colonne est une colonne à soutirage latéral et l'ADAME purifié est soutiré dans le tiers inférieur de la colonne.

L'alimentation de la première colonne a la composition massique et les caractéristiques suivantes :
ADAME : 48% - AE : 23% - DMAE : 14%
APA : 1% - EPA : 0,2% - EPE : 0,04% - Autres lourds : 164 ppm, catalyseur : 1,5%
Flux total : 6780 kg/h - température : 110 °C - Pression : 0,891 bar (81.9 kPa)

Une simulation ASPEN à l'aide d'un modèle thermodynamique a été réalisée et fournit la composition massique suivante pour le produit purifié obtenu en soutirage latéral.
ADAME : 99,8% - DMAE : < 1 ppm
APA traces - EPA : 510 ppm - EPE : 936 ppm - Lourds dont EPA : 534 ppm
Flux total : 2870 kg/h - Température : 120°C - Pression : 0,188 bar (18.8 kPa)

Dans cette configuration, l'ADAME est récupéré avec un rendement de l'ordre de 88% par rapport au flux d'alimentation, et l'ADAME a une pureté supérieure à 99,8% .La consommation énergétique globale est de 1,54 MW. La somme des impuretés lourdes (EPE, EPA, autres lourds) s'élève à 1470 ppm.

### Exemple 3 (selon l'invention)

Une simulation ASPEN à l'aide d'un modèle thermodynamique a été réalisée sur le mélange réactionnel brut d'ADAME, tel que décrit dans les exemples 1 et 2, mais soumis à une purification à l'aide du système de purification tel que représenté sur la Figure.

Dans cet exemple, la colonne à partition est stabilisée au niveau du condenseur de tête par de la PTZ et l'ADAME soutiré latéralement en phase gazeuse est stabilisé par de l'EMHQ.

Dans cette configuration, le nombre de plateaux des différentes sections est comme suit : N1 : 8 - N2 : 6 - N3 : 5- N4 : 10 - N5 : 1.

L'énergie fournie par le bouilleur est 1,4 MW

Le produit purifié soutiré latéralement a la composition massique suivante :
ADAME : 99,8% - DMAE : traces
APA traces - EPA : 237 ppm - EPE : 919 ppm - Lourds dont EPA : 254ppm
Flux total : 2870 kg/h - Température : 110°C - Pression : 0,118 bar (11.8 kPa)

Dans cette configuration, l'ADAME est récupéré avec un rendement de l'ordre de 88% par rapport au flux d'alimentation, et l'ADAME a une pureté supérieure à 99,8%.

La consommation énergétique globale est de 1,4 MW. La somme des impuretés lourdes (EPE, EPA, autres lourds) s'élève à 1173 ppm.

Par rapport au procédé de purification de l'art antérieur utilisant 2 ou 3 colonnes de distillation, la chaleur nécessaire pour purifier le flux réactionnel est réduite de l'ordre de 18% (schéma à 3 colonnes) ou de 10% (schéma à 2 colonnes).

La teneur en impuretés lourdes diminue aussi significativement de l'ordre de 20 à 30%.

### Exemple 4 : Dégradation du produit de pied lors de la distillation

Le but de cet exemple est de montrer l'importance du positionnement de la paroi séparatrice dans la colonne à partition pour la qualité du produit obtenu en soutirage latéral, en particulier la nécessité d'avoir une paroi séparant la partie basse de la colonne. Nous avons utilisé un montage de laboratoire composé d'un réacteur agité, d'une colonne à distiller et d'un bain d'huile permettant de chauffer le produit à la température désirée. Un mélange ayant une composition proche de la composition attendue en pied de la colonne de distillation au niveau du bouilleur B1 a été introduit dans le réacteur et soumis à une température de 140°C à P atm pendant 3 heures.

Le distillat recueilli en tête de la colonne à distiller et le pied de distillation ont ensuite été analysés.

Le tableau ci-après présente les quantités massiques des produits légers distillés comparativement aux quantités présentes dans le mélange introduit dans le réacteur.

| | Alimentation | distillat | Commentaires |
|---|---|---|---|
| EtOH, g | 0 | 0,086 | Formation |
| AE, g | 0 | 0,145 | Formation |
| DMAE, g | 0 | 10,87 | Formation |
| EPE, g | 0 | 0,706 | Formation |

Cet essai montre que le produit de pied de la colonne de distillation du fait de la présence de catalyseur évolue et forme des composés légers lorsqu'on le maintient en température.

Selon l'invention, une paroi séparatrice allant jusqu'en bas de la colonne permet d'éviter que ces produits légers polluent le soutirage latéral.

## Revendications

1. Procédé de récupération d'un ester (méth)acrylique en C₄-C₁₂ purifié, à partir d'un mélange réactionnel brut obtenu par transestérification d'un ester (méth)acrylique léger par l'alcool correspondant, ledit mélange réactionnel brut comprenant un catalyseur de transestérification,
ledit procédé étant **caractérisé en ce qu'**il est mis en œuvre à l'aide d'un système de purification comprenant une colonne à partition équipée d'une paroi séparatrice créant des zones de séparation dans la colonne, la paroi étant non jointive avec le dôme supérieur de la colonne en partie haute et jointive avec le fond de la colonne en partie basse, ladite colonne à partition associée en tête à un condenseur unique et en pied à deux bouilleurs, comprenant une section commune de rectification au-dessus de la paroi, une section de préfractionnement comportant l'alimentation de la colonne, une section de soutirage séparée de la section de préfractionnement par la paroi comprenant le soutirage de l'ester purifié, et
**caractérisé en ce que** i) un flux gazeux est extrait en tête de la section de rectification et recyclé après condensation au moins en partie dans le réacteur, ii) un flux est soutiré en pied de la section de préfractionnement et recyclé au moins en partie dans le réacteur, iii) un flux est soutiré en pied de la section de soutirage et recyclé au moins en partie dans la section de préfractionnement de la colonne, et iv) un flux d'ester (méth)acrylique purifié est soutiré latéralement de la section de soutirage en un point situé au-dessus du soutirage de pied de ladite section de soutirage.

2. Procédé selon la revendication 1 **caractérisé en ce que** l'alcool est un alcool aliphatique primaire ou secondaire, comportant une chaine alkyle linéaire ou ramifiée comportant de 4 à 12 atomes de carbone, de préférence de 5 à 10 atomes de carbone, et pouvant être interrompue par un ou plusieurs hétéroatomes tels que N ou O, de préférence N.

3. Procédé selon la revendication 1 ou 2 **caractérisé en ce que** l'alcool est un alcool aliphatique primaire ou secondaire, à chaine alkyle linéaire ou ramifiée comportant de 4 à 12 atomes de carbone, de préférence de 5 à 10 atomes de carbone.

4. Procédé selon la revendication 3 **caractérisé en ce que** l'alcool est le 2-éthyl hexanol, le 2-octanol, ou le 2-propyl heptanol, de préférence le 2-octanol.

5. Procédé selon la revendication 1 ou 2 **caractérisé en ce que** l'alcool est un dialkyl amino alcool, de formule :
HO-A-N (R₁)(R₂)
dans laquelle
- A est un radical alkylène, linéaire ou ramifié, en C₁-C₅
- R₁ et R₂, identiques ou différents l'un de l'autre, représentent chacun un radical alkyle en C₁-C₄.

6. Procédé selon la revendication 5 **caractérisé en ce que** l'alcool est le N,N-diméthyl aminoéthanol, le N,N-diéthyl aminoéthanol, le N,N-diméthyl aminopropanol, de préférence, le N,N-diméthyl aminoéthanol.

7. Procédé selon l'une quelconque des revendications précédentes **caractérisé en ce que** l'ester (méth)acrylique léger est le (méth)acrylate de méthyle ou le (méth)acrylate d'éthyle, de préférence l'acrylate d'éthyle.

8. Procédé selon l'une quelconque des revendications précédentes **caractérisé en ce que** le nombre d'étages théoriques de la section de rectification est compris entre 1 et 15.

9. Procédé selon l'une quelconque des revendications précédentes **caractérisé en ce que** le nombre d'étages théoriques de la section de préfractionnement est compris entre 1 et 20.

10. Procédé selon l'une quelconque des revendications précédentes **caractérisé en ce que** le nombre d'étages théoriques de la section de soutirage est compris entre 1 et 15.

11. Procédé selon l'une quelconque des revendications précédentes **caractérisé en ce que** la stabilisation du système de purification est réalisée à l'aide d'un inhibiteur de polymérisation unique, de préférence injecté au niveau du condenseur de tête, l'ester (méth)acrylique purifié étant soutiré latéralement de la colonne à partition sous forme d'un flux liquide ou gazeux stabilisé.

12. Procédé selon l'une quelconque des revendications 1 à 10 **caractérisé en ce que** la stabilisation du système de purification est réalisée à l'aide d'un premier inhibiteur de polymérisation, de préférence injecté au niveau du condenseur de tête, l'ester (méth)acrylique purifié étant soutiré latéralement de la colonne à partition sous forme d'un flux gazeux qui, après condensation, est ensuite stabilisé avec un inhibiteur de polymérisation différent du premier inhibiteur.

13. Procédé selon l'une quelconque des revendications précédentes **caractérisé en ce que** l'ester (méth)acrylique en C₄-C₁₂ purifié est l'acrylate de diméthyl aminoéthyle ou l'acrylate de 2- octyle.

14. Procédé de production d'un ester (méth)acrylique en C₄-C₁₂ purifié, par transestérification d'un ester (méth)acrylique léger par l'alcool correspondant **caractérisé en ce que** le mélange réactionnel brut est soumis au procédé de récupération tel que défini selon l'une quelconque des revendications précédentes.

15. Utilisation d'un système de purification pour récupérer un ester (méth)acrylique en C₄-C₁₂ purifié, à partir d'un mélange réactionnel brut obtenu par transestérification catalytique d'un ester (méth)acrylique léger par l'alcool correspondant, ledit système comprenant une colonne à partition équipée d'une paroi séparatrice créant des zones de séparation dans la colonne, la paroi étant non jointive avec le dôme supérieur de la colonne en partie haute et jointive avec le fond de la colonne en partie basse, ladite colonne à partition associée en tête à un condenseur unique et en pied à deux bouilleurs, comprenant une section commune de rectification au-dessus de la paroi, une section de préfractionnement comportant l'alimentation de la colonne en un mélange à purifier, une section de soutirage séparée de la section de préfractionnement par la paroi comprenant le soutirage du produit purifié.

## Patentansprüche

1. Verfahren zur Rückgewinnung eines aufgereinigten (Meth)acrylsäure-C₄-C₁₂-esters, ausgehend von einer unbehandelten Reaktionsmischung, welche durch Umesterung eines leichtflüchtigen (Meth)acrylsäureesters mit dem entsprechenden Alkohol erhalten wurde, wobei die unbehandelte Reaktionsmischung einen Umesterungskatalysator umfasst,
wobei das Verfahren **dadurch gekennzeichnet ist, dass** es mit Hilfe eines Aufreinigungssystems durchgeführt wird, welches eine unterteilte Kolonne umfasst, die mit einer Trennwand ausgestattet ist, welche in der Kolonne getrennte Bereiche schafft, wobei die Wand im oberen Abschnitt nicht an die obere Kuppel der Kolonne angefügt ist und sie im unteren Abschnitt an den Boden der Kolonne angefügt ist, wobei die unterteilte Kolonne kopfseitig mit einem einzigen Verflüssiger und sumpfseitig mit zwei Verdampfern in Verbindung steht, wobei sie eine gemeinsame Rektifikationssektion, welche sich oberhalb der Wand befindet, eine Vorfraktionierungssektion, welche die Speiseleitung der Kolonne aufweist, eine Entnahmesektion umfasst, wobei letztere durch die Wand von der Vorfraktionierungssektion getrennt ist und sie die Entnahmeleitung für den aufgereinigten Ester umfasst, und
es **dadurch gekennzeichnet ist, dass** i) ein gasförmiger Stoffstrom kopfseitig aus der Rektifikationssektion abgeführt wird und, nachdem er zumindest teilweise verflüssigt wurde, in den Reaktor zurückgeführt wird, ii) ein Stoffstrom sumpfseitig aus der Vorfraktionierungssektion entnommen und zumindest teilweise in den Reaktor zurückgeführt wird, iii) ein Stoffstrom sumpfseitig aus der Entnahmesektion entnommen und zumindest teilweise in die Vorfraktionierungssektion zurückgeführt wird, und iv) ein Stoffstrom an aufgereinigtem (Meth)acrylsäureester seitlich aus der Entnahmesektion entnommen wird, an einer Stelle, die sich oberhalb sumpfseitigen Entnahmeleitung der Entnahmesektion befindet.

2. Verfahren nach Anspruch 1, **dadurch gekennzeichnet, dass** es sich bei dem Alkohol um einen primären oder sekundären aliphatischen Alkohol handelt, der eine geradkettige oder verzweigte Alkylkette aufweist, welche 4 bis 12 Kohlenstoffatome, vorzugsweise 5 bis 10 Kohlenstoffatome aufweist, wobei sie weiterhin von einem oder mehreren Heteroatomen wie etwa N oder O, vorzugsweise N, unterbrochen sein kann.

3. Verfahren nach Anspruch 1 oder 2, **dadurch gekennzeichnet, dass** es sich bei dem Alkohol um einen primären oder sekundären aliphatischen Alkohol handelt, mit einer geradkettigen oder verzweigten Alkylkette, welche 4 bis 12 Kohlenstoffatome, vorzugsweise 5 bis 10 Kohlenstoffatome aufweist.

4. Verfahren nach Anspruch 3, **dadurch gekennzeichnet, dass** es sich bei dem Alkohol um 2-Ethylhexanol, 2-Octanol oder 2-Propylheptanol, vorzugsweise 2-Octanol, handelt.

5. Verfahren nach Anspruch 1 oder 2, **dadurch gekennzeichnet, dass** es sich bei dem Alkohol um einen Dialkylaminoalkohol mit der folgenden Formel handelt:
HO-A-N (R₁)(R₂)
wobei
- A ein geradkettiger oder verzweigter C₁-C₅-Alkylenrest ist,
- R₁ und R₂, die gleichartig oder verschiedenartig sein können, jeweils für einen C₁-C₄-Alkylrest stehen.

6. Verfahren nach Anspruch 5, **dadurch gekennzeichnet, dass** es sich bei dem Alkohol um N,N-Dimethylaminoethanol, N,N-Diethylaminoethanol, N,N-Dimethylaminopropanol, vorzugsweise um N,N-Dimethylaminoethanol, handelt.

7. Verfahren nach einem beliebigen der vorhergehenden Ansprüche, **dadurch gekennzeichnet, dass** es sich bei dem leichtflüchtigen (Meth)acrylsäureester um Methyl(meth)acrylat, Ethyl(meth)acrylat, vorzugsweise um Ethylacrylat, handelt.

8. Verfahren nach einem beliebigen der vorhergehenden Ansprüche, **dadurch gekennzeichnet, dass** die Anzahl an theoretischen Böden der Rektifikationssektion im Bereich von 1 bis 15 liegt.

9. Verfahren nach einem beliebigen der vorhergehenden Ansprüche, **dadurch gekennzeichnet, dass** die Anzahl an theoretischen Böden der Vorfraktionierungssektion im Bereich von 1 bis 20 liegt.

10. Verfahren nach einem beliebigen der vorhergehenden Ansprüche, **dadurch gekennzeichnet, dass** die Anzahl an theoretischen Böden der Entnahmesektion im Bereich von 1 bis 15 liegt.

11. Verfahren nach einem beliebigen der vorhergehenden Ansprüche, **dadurch gekennzeichnet, dass** die Stabilisierung des Aufreinigungssystems mit Hilfe eines einzigen Polymerisationshemmers erfolgt, welcher vorzugsweise auf Höhe des kopfseitigen Verflüssigers eingeleitet wird, wobei der aufgereinigte (Meth)acrylsäureester in Form eines stabilisierten flüssigen oder gasförmigen Stoffstroms seitlich aus der unterteilten Kolonne entnommen wird.

12. Verfahren nach einem beliebigen der Ansprüche 1 bis 10, **dadurch gekennzeichnet, dass** die Stabilisierung des Aufreinigungssystems mit Hilfe eines einzigen Polymerisationshemmers erfolgt, welcher vorzugsweise auf Höhe des kopfseitigen Verflüssigers eingeleitet wird, wobei der aufgereinigte (Meth)acrylsäureester in Form eines gasförmigen Stoffstroms seitlich aus der unterteilten Kolonne entnommen wird, welcher anschließend, nach der Verflüssigung, mit einem Polymerisationshemmer stabilisiert wird, der sich vom ersten Hemmer unterscheidet.

13. Verfahren nach einem beliebigen der vorhergehenden Ansprüche, **dadurch gekennzeichnet, dass** es sich bei dem aufgereinigten C₄-C₁₂- (Meth) acrylsäureester um Dimethylaminoethylacrylat oder 2-Octylacrylat handelt.

14. Verfahren zur Produktion eines aufgereinigten C₄-C₁₂-(Meth)acrylsäureesters, durch Umesterung eines leichtflüchtigen (Meth)acrylsäureesters mit dem entsprechenden Alkohol, **dadurch gekennzeichnet, dass** die unbehandelte Reaktionsmischung dem Rückgewinnungsverfahren gemäß der Begriffsbestimmung in einem beliebigen der vorhergehenden Ansprüche unterzogen wird.

15. Verwendung eines Aufreinigungssystems, um einen aufgereinigten (Meth)acrylsäure-C₄-C₁₂-ester ausgehend von einer unbehandelten Reaktionsmischung zurückzugewinnen, welche durch katalytische Umesterung eines leichtflüchtigen (Meth)acrylsäureesters mit dem entsprechenden Alkohol erhalten wurde, wobei das System eine unterteilte Kolonne umfasst, die mit einer Trennwand ausgestattet ist, welche in der Kolonne getrennte Bereiche schafft, wobei die Wand im oberen Abschnitt nicht an die obere Kuppel der Kolonne angefügt ist und sie im unteren Bereich an den Boden der Kolonne angefügt ist, wobei die unterteilte Kolonne kopfseitig mit einem einzigen Verflüssiger und sumpfseitig mit zwei Verdampfern in Verbindung steht, wobei sie eine gemeinsame Rektifikationssektion, welche sich oberhalb der Wand befindet, eine Vorfraktionierungssektion, welche die Einspeisung einer aufzureinigenden Mischung in die Kolonne aufweist, eine Entnahmesektion umfasst, wobei letztere durch die Wand von der Vorfraktionierungssektion getrennt ist und die Entnahmeleitung für das aufgereinigte Produkt umfasst.

## Claims

1. A process for the recovery of a purified C₄-C₁₂ (meth)acrylic ester, from a crude reaction mixture obtained by transesterification of a light (meth)acrylic ester by the corresponding alcohol, said reaction mixture comprising a catalyst for the transesterification reaction,
said process being **characterized in that** it is carried out using a purification system comprising a divided wall column equipped with a separating wall creating separation zones in the column, the wall not being joined to the upper dome of the column in the top part and being joined to the bottom of the column in the bottom part, said divided wall column combined at the top with a single condenser and at the bottom with two boilers, comprising a common rectification section above the wall, a prefractionation section comprising the feeding of the column, a withdrawal section separated from the prefractionation section by the wall comprising the withdrawal of the purified ester, and
**characterized in that** i) a gas stream is extracted at the top of the rectification section and recycled after condensation at least in part in the reactor, ii) a stream is withdrawn at the bottom of the prefractionation section and recycled at least in part in the reactor, iii) a stream is withdrawn at the bottom of the withdrawal section and recycled at least in part in the prefractionation section of the column, and iv) a stream of purified (meth)acrylic ester is drawn off as a sidestream from the withdrawal section at a point located above the bottom withdrawal of said withdrawal section.

2. The process as claimed in claim 1, **characterized in that** the alcohol is a primary or secondary aliphatic alcohol comprising a linear or branched alkyl chain comprising from 4 to 12 carbon atoms, preferably from 5 to 10 carbon atoms, and being able to be interrupted by one or more heteroatoms, such as N or O, preferably N.

3. The process as claimed in claim 1 or 2, **characterized in that** the alcohol is a primary or secondary aliphatic alcohol, having a linear or branched alkyl chain comprising from 4 to 12 carbon atoms, preferably from 5 to 10 carbon atoms.

4. The process as claimed in claim 3, **characterized in that** the alcohol is 2-ethylhexanol, 2-octanol or 2-propylheptanol, preferably 2-octanol.

5. The process as claimed in claim 1 or 2, **characterized in that** the alcohol is a dialkylaminoalcohol, of formula:
HO-A-N (R₁)(R₂)
in which
- A is a linear or branched C₁-C₅ alkylene radical,
- R₁ and R₂, which are identical to or different from each other, each represent a C₁-C₄ alkyl radical.

6. The process as claimed in claim 5, **characterized in that** the alcohol is N,N-dimethylaminoethanol, N,N-diethylaminoethanol or N,N-dimethylaminopropanol, preferably N,N-dimethylaminoethanol.

7. The process as claimed in any one of the preceding claims, **characterized in that** the light (meth)acrylic ester is methyl (meth)acrylate or ethyl (meth)acrylate, preferably ethyl acrylate.

8. The process as claimed in any one of the preceding claims, **characterized in that** the number of theoretical stages of the rectification section is between 1 and 15.

9. The process as claimed in any one of the preceding claims, **characterized in that** the number of theoretical stages of the prefractionation section is between 1 and 20.

10. The process as claimed in any one of the preceding claims, **characterized in that** the number of theoretical stages of the withdrawal section is between 1 and 15.

11. The process as claimed in any one of the preceding claims, **characterized in that** the purification system is stabilized using a single polymerization inhibitor, preferably injected at the top condenser, the purified (meth)acrylic ester being withdrawn as a sidestream from the divided wall column in the form of a stabilized liquid or gas stream.

12. The process as claimed in any one of claims 1 to 10, **characterized in that** the purification system is stabilized using a first polymerization inhibitor, preferably injected at the top condenser, the purified (meth)acrylic ester being withdrawn as a sidestream from the divided wall column in the form of a gas stream which, after condensation, is subsequently stabilized with a different polymerization inhibitor from the first inhibitor.

13. The process as claimed in any one of the preceding claims, **characterized in that** the purified C₄-C₁₂ (meth)acrylic ester is dimethylaminoethyl acrylate or 2-octyl acrylate.

14. A process for the production of a purified C₄-C₁₂ (meth)acrylic ester, by transesterification of a light (meth)acrylic ester with the corresponding alcohol, **characterized in that** the crude reaction mixture is subjected to the recovery process as claimed in any one of the preceding claims.

15. The use of a purification system for recovering a purified C₄-C₁₂ (meth)acrylic ester, starting from a crude reaction mixture obtained by catalytic transesterification of a light (meth)acrylic ester by the corresponding alcohol, said system comprising a divided wall column equipped with a separating wall creating separation zones in the column, the wall not being joined to the upper dome of the column in the top part and being joined to the bottom of the column in the bottom part, said divided wall column combined at the top with a single condenser and at the bottom with two boilers, comprising a common rectification section above the wall, a prefractionation section comprising the feeding of the column with a mixture to be purified, a withdrawal section separated from the prefractionation section by the wall comprising the withdrawal of the purified product.
